# EUROPEAN PATENT APPLICATION

(11) **EP 1 060 758 A1**
(43) Date of publication of application: **20.12.2000**
(21) Application number: 00111931.2
(22) Date of filing: 15.06.2000
(51) Int. Cl.: A61M 25/10

(54) **Aortic occlusion cannula**

(30) Priority: 15.06.1999 US 333804
(71) Applicant: MEDTRONIC, INC., Minneapolis, Minnesota 55432-3576 (US)
(72) Inventor: Wiersma, Jeffery, Hudsonville, Michigan 49426 (US); Booth, William M., Paw Paw, Michigan 49079 (US); Rom, Paul F., Kentwood, Michigan 49508 (US); Vroegop, Eric, Holland, Michigan 49423 (US)
(74) Representative: Hughes, Andrea Michelle

(57) **Abstract**

A multi-lumen aortic occlusion balloon cannula employed to occlude the aorta and provide oxygenated blood to a patient's arterial system during cardiac surgery. The cannula 10 includes a cardioplegia delivery lumen 44 for the delivery of cardioplegia solution, a blood lumen 40 for delivery of oxygenated blood into the patient's aorta, a blood pressure lumen 46 for pressure sensing, and a lumen 42 for inflation and deflation of an asymmetric balloon 20 to occlude the aorta at a location distal to the delivery of oxygenated blood. The asymmetric balloon is shaped to expand more on a first side 32 of the cannula body than on a second side 36 opposite to the first side to displace the first side away from the side of the aorta that it bears against when inflated. A plurality of blood outlet ports 30₁ -30_{N} proximal to the balloon are arranged generally in a pattern extending along the first side such that at least a distal sub-set thereof are disposed away from that side of the aorta when the balloon is inflated. The distal cannula portion 14 can be advantageously rotated within the aorta and arterial vessel it extends through to align the plane of asymmetric expansion of the balloon and direction of blood injection along the first side of the cannula body to a feature of the aorta or arterial vessel that it passes through. The increased distance between the aorta or arterial vessel wall or other vascular wall and the blood outlet ports effected by the more pronounced expansion of the balloon along the first side of the cannula body reduces the force of the blood jets emitted from the blood outlet ports and striking the aorta or arterial vessel. The velocity and force of the blood jets is attenuated by resistance of movement of blood already present in the vessel lumen, and the attenuation increases with distance. Consequently, injury to the vascular wall and the likelihood of dislodging plaque is also advantageously diminished.

## Description

This invention relates to blood vessel occlusion cannulas, particularly multi-lumen, aortic occlusion balloon cannulas employed to occlude the aorta and provide oxygenated blood to a patient's arterial system during cardiac surgery.

Cannulas are often used during cardiac surgery to conduct fluid to and from the various chambers and nearby vessels of the heart. One desirable goal of cardiac surgery as a discipline is to minimize the number of incisions that are formed in the blood vessels near the heart. It is especially important to minimize the number of incisions in the aorta in view of the significant fluid pressures that are experienced by this vessel during normal beating of the heart.

Multi-lumen, balloon bearing, aortic occlusion cannulas used in cardiopulmonary bypass procedures for providing arterial perfusion with oxygenated blood while the heart is stopped to facilitate cardiac bypass surgery are disclosed in U.S. Patent Nos. 5,312,344 and 5,879,499, for example. The distal cannula portion of this aortic occlusion cannula body supports an inflatable occlusion balloon that can be located within the ascending aorta between the coronary ostium and the brachiocephalic trunk to close off the aorta upon inflation of the balloon. The cannula body encloses a number of cannula lumens extending distally from the proximal cannula portion disposed outside the patient's body to the distal cannula portion. A balloon inflation lumen extends into the balloon lumen to allow it to be inflated in situ with saline solution and deflated for withdrawal of the cannula. Cardioplegia solutions can be delivered through a cardioplegia lumen and emitted from a cardioplegia outlet port distal to the balloon into the coronary arteries. Blood pressure measurements are taken through a blood pressure measurement lumen extending to a blood pressure port distal to the inflated balloon. A blood delivery or blood lumen extends to one or more blood outlet ports through the cannula wall proximal to the balloon.

In use, with the distal cannula portion situated with respect to the aorta and the balloon inflated, venous blood is withdrawn from the patient's circulatory system employing a further cannula. The withdrawn venous blood is oxygenated externally to the body and returned through the blood lumen under pressure and emitted through the blood outlet port into the aorta and the vessel that the cannula is inserted through proximal to the inflated occlusion balloon. The oxygenated blood is then distributed through the arteries branching from the aorta and the patient's arterial circulatory system. Such multi-lumen, balloon bearing aortic occlusion cannulas limit or eliminate incisions directly into the aorta wall because they can be introduced percutaneously at a site of an artery remote from the aorta, easily advanced to locate the distal cannula portion at the above-described site, and then withdrawn after the surgical procedure is completed.

Another desirable goal of arterial perfusion is to minimize trauma to the heart and nearby vessel walls occasioned by use of the cannula. It is desirable to minimize the cannula diameter to facilitate introduction and advancement through the arterial vessels. But, it must also provide as large a blood lumen as is practical while also accommodating the further blood pressure sensing, cardioplegia delivery and balloon inflation lumens while retaining structural integrity and desirable handling characteristics. The volume of oxygenated blood that must be delivered at the blood pressure necessary to sustain the patient's blood circulation necessitates providing as large cross-section area blood lumen as possible and one or more blood outlet ports having a total outlet area that is as large as is practical without structurally jeopardizing the integrity of the outer sheath of the cannula. A plurality of blood injection or blood outlet ports are formed from the blood lumen through the cannula outer sheath enclosing the blood lumen for a blood injection distance extending proximally to the balloon. The blood outlet ports are arranged in parallel lines in a pattern extending around the circumference of the cannula outer sheath and equally spaced apart.

When the blood is injected back into the aorta superior to the balloon, it is injected as a high pressure jet of blood that can strike the wall of the aorta or the vessel that the cannula extends through to access the aorta at a velocity and with a force that can be injurious to the wall of the aorta or the vessel. In some patients, the fluid flow may dislodge plaque from the aorta wall or arterial vessel wall or other vascular wall surrounding the cannula, thereby increasing the risk of embolic complications. There is a need to minimize the possibility of such injuries to vascular walls and complications of use of an arterial cannula.

The invention that accomplishes this need provides a medical cannula for conducting oxygenated blood from a source exterior to a patient's body to a site in a patient's arterial vascular system, the cannula extending between a proximal cannula portion for receiving the oxygenated blood and a distal cannula portion for injecting the oxygenated blood into the site adjacent to a vascular wall, said cannula further comprising:
an elongated cannula body extending distally from a proximal cannula body end in the proximal cannula portion to a distal cannula body end in the distal cannula portion, the cannula body formed of a tubular outer sheath enclosing a blood delivery lumen and a balloon inflation lumen extending from said proximal cannula portion to said distal cannula portion and having a cannula body longitudinal axis;
an inflatable, asymmetric balloon formed in said distal cannula portion and coupled with said balloon inflation lumen, said asymmetric balloon shaped asymmetrically with respect to the axis of the elongated cannula body to expand a first distance away from a first side of the distal cannula portion and a second distance away from a second side of the distal cannula portion when inflated, the first distance exceeding the second distance, whereby the inflation of the balloon displaces the first and second sides of the distal cannula portion away from a vascular wall that the balloon bears against when inflated, and the displacement of the first side of the distal cannula portion exceeds the displacement of the second side of the distal cannula portion; and
at least one blood outlet port extending from said blood lumen and through said first side of said tubular outer sheath in said distal cannula portion such that the increased displacement of the blood outlet port from the vascular wall caused by asymmetric expansion of the balloon away from the first side of the cannula body reduces the force of blood jets emitted from the blood outlet ports and striking the vascular wall spaced therefrom when oxygenated blood is injected through the blood lumen from the proximal cannula portion. In one form, the invention comprises an arterial cannula for occluding the vascular system during cardiac surgery which possesses an arrangement of blood outlet ports and an asymmetric balloon formed in a distal portion of the cannula body which maximizes the distance between the blood outlet port or ports through which oxygenated blood is injected at highest velocity and the vascular wall, so that the force of the injected, oxygenated blood striking the same is minimized.

In a particular embodiment, in the context of an aortic occlusion cannula, the asymmetric balloon is shaped to expand outward a greater distance from a first side of the cannula body than it expands outward from a second side of the cannula body opposite to the first side, to displace the first side of the cannula body away from the side of the aorta wall that the balloon bears against when inflated. The blood outlet port or plurality of blood outlet ports proximal to the balloon are arranged generally in a pattern extending along the first side of the cannula body such that they are disposed away from the aorta wall by the expanded balloon. The pattern can comprise one or more sets or lines of blood outlets formed through the cannula outer sheath and the blood lumen and extending proximally from the balloon for a blood injection segment length along the first side of the cannula. Two or more parallel lines of equidistantly spaced apart blood outlet ports are preferred to maximize blood flow, wherein the centers of the blood outlet ports of one line are staggered with respect to the centers of the blood outlet ports of the second line so that sufficient distance can be maintained between each blood outlet port to avoid unduly weakening the outer sheath of the cannula body. In this case, the multiple lines of blood fluid ports are symmetrically arranged with respect to the plane of asymmetric expansion of the balloon extending through the axis of the cannula body and intersecting the first and second sides thereof.

The distal cannula portion can be advantageously rotated within the aorta and arterial vessel it extends through to align the plane of asymmetric expansion of the balloon and direction of blood injection along the first side of the cannula body to a feature of the aorta or arterial vessel that it passes through. The increased distance between the aorta wall and the blood outlet ports effected by the more pronounced expansion of the balloon along the first side of the cannula body reduces the force of the blood jets emitted from the blood outlet ports and striking the aorta or arterial vessel. The velocity and force of the blood jets is attenuated by resistance of movement of blood already present in the vessel lumen, and the attenuation increases with distance. Consequently, injury to the vessel walls and the likelihood of dislodging plaque is also advantageously diminished.

These and other advantages and features of the present invention will become apparent from the preferred embodiments thereof, given by way of example only, depicted in the drawing figures wherein:
FIG. 1 depicts a multi-lumen aortic occlusion cannula in which the various embodiments of the invention can be incorporated;
FIG. 2 is an expanded view of the distal portion of the aortic occlusion cannula of FIG. 1 showing the orientation of the inflated asymmetric balloon and a line of the blood outlet ports in the blood injection segment of the distal cannula portion in accordance with one embodiment of the invention;
FIG. 3 is a cross-section view of the cannula body taken along lines 3-3 of FIG. 2 depicting the internal arrangement of cannula lumens and the line of blood outlet ports with respect to the plane of asymmetric expansion of the balloon extending through the axis of the cannula body ;
FIG. 4 is an expanded view of the distal portion of the aortic occlusion cannula of FIG. 1 showing the orientation of the inflated asymmetric balloon and two lines of the blood outlet ports in the blood injection segment of the distal cannula portion in accordance with one embodiment of the invention;
FIG. 5 is a cross-section view of the cannula body taken along lines 5-5 of FIG. 4 depicting the internal arrangement of cannula lumens and the line of blood outlet ports with respect to the plane of asymmetric expansion of the balloon extending through the axis of the cannula body ;
FIG. 6 is a view of the aortic occlusion cannula percutaneously inserted into a patient's arterial system to locate the distal cannula portion in a desired relation to the aorta and of an ultrasound probe for viewing the distal cannula portion to aid in rotating it to a desired orientation of the blood outlet ports for injection of oxygenated blood;
FIG. 7 is an expanded view of the distal cannula portion and ultrasound probe of FIG. 6;
FIG. 8 is a schematic view of the injection of blood from the plurality of blood outlet ports of the embodiments of FIGs. 1-3 and 4-5.

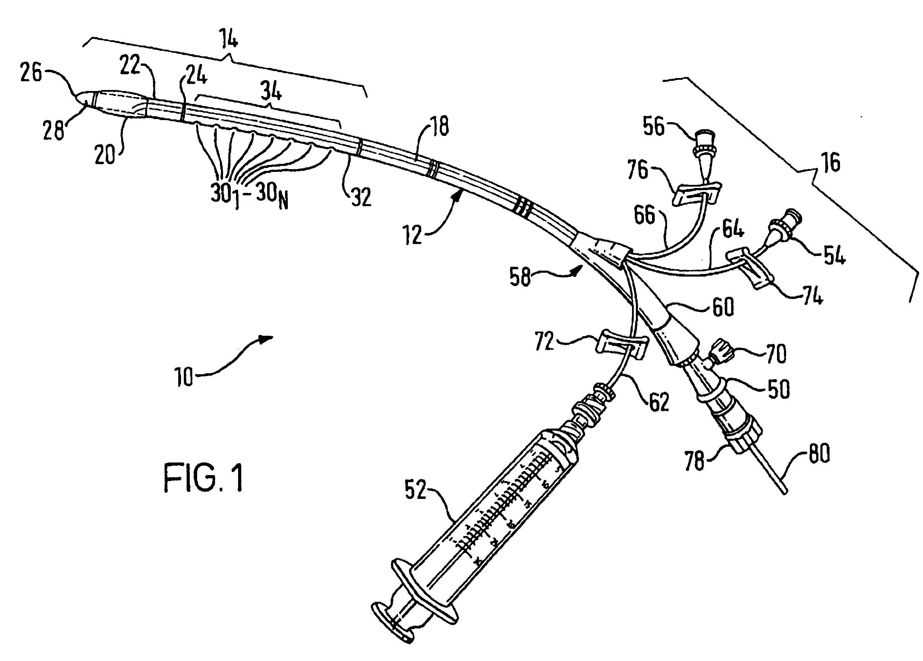

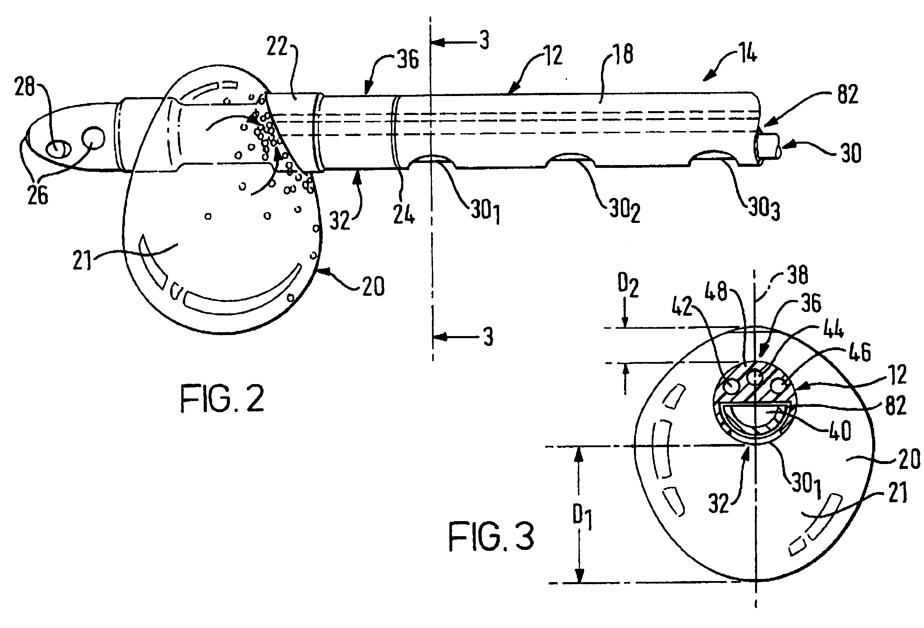

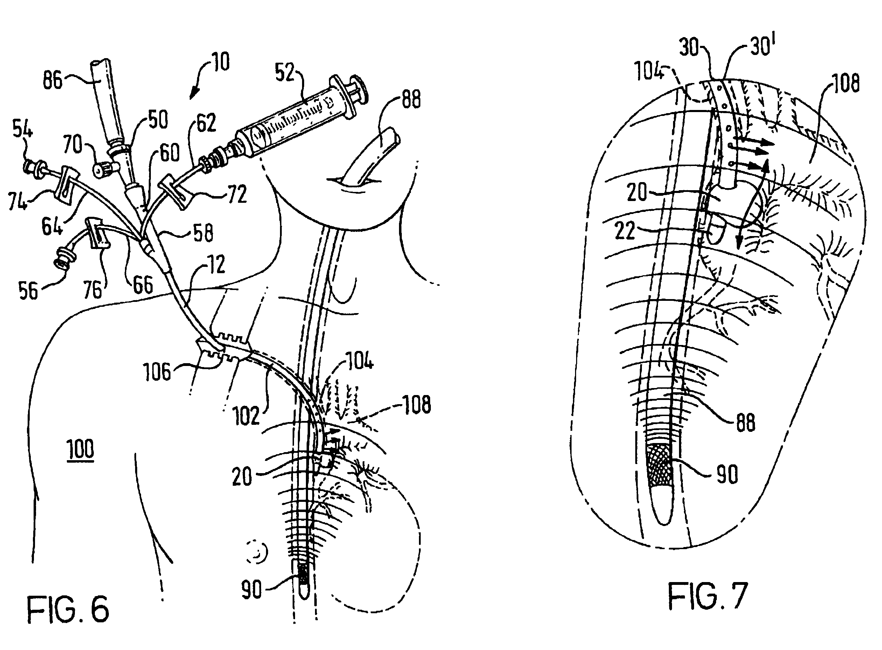

FIG. 1 depicts a multi-lumen, aortic occlusion cannula 10 in which the various embodiments of the invention shown in FIGs. 2-5 can be incorporated and which can be used as shown in FIGs. 6 and 7. The multi-lumen, aortic occlusion cannula 10 is formed with an elongated cannula body 12 extending between a distal cannula portion 14 bearing a balloon 20 and a proximal cannula portion 16. The cannula body 12 comprises a tubular outer sheath 18 and a distal tip member 22 formed of silicone rubber or polyvinyl chloride, for example, in a diameter of about 6.7-7.3 mm (20-22 French). The cannula body 12 encloses a plurality of lumens that are accessed by separate components of the proximal cannula portion 16 and are coupled with ports in the distal cannula portion 14 as described below.

The distal cannula portion 14 includes the distal tip member 22 having a tip member proximal end fitted to the cannula body distal end at attachment junction 24 and a distal section of the tubular outer sheath 18. The distal tip member 22 supports an inflatable, asymmetric occlusion balloon 20 that can be located within the ascending aorta between the lower portion of the aortic valve and its coronary ostium and the brachiocephalic trunk and inflated to close off the aorta lumen. At least one, but preferably a plurality N, blood outlet ports 30₁ - 30_{N} are formed from the blood lumen 40 through the tubular outer sheath 18 extending along a first side 32 thereof The spaced apart blood outlet ports 30₁ -30_{N} extend proximally of the balloon 20 and the attachment junction 24 for a blood injection segment length 34 along the first side 32. The blood outlet ports 30₁ - 30_{N} are arranged generally in a pattern, e.g., a blood outlet line 30 as shown in FIGs. 1-3.

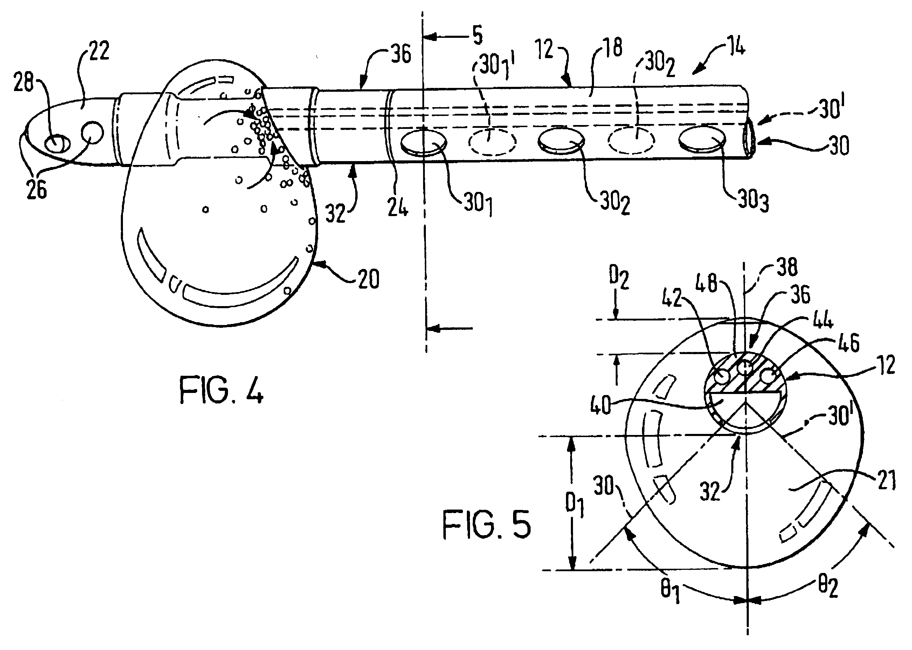

Preferably as many blood outlet ports as possible are formed through the tubular outer sheath 18 and spaced apart along the blood segment length 34 without impairing the strength of the tubular outer sheath 18 or the ability to advance the distal cannula portion over a guide wire and into the desired position in the aorta. Two parallel, staggered or offset, blood outlet lines 30 and 30' are shown in FIGs. 4 and 5 comprising N blood outlet ports 30₁ - 30_{N} and N' blood outlet ports 30'₁ -30'_{N'}, respectively, formed through the cannula outer sheath 18 and in fluid communication with blood lumen 40 (shown in FIG. 5) enclosed therein.

In each embodiment illustrated in FIGs. 3 and 5, the blood outlet lines 30 alone or 30 and 30' considered together are preferably parallel to the longitudinal axis of the cannula body 12 and are symmetrically arranged with respect to the asymmetric balloon 20 and a blood injection plane 38 as described further below.

With respect to the cannula body 12, FIGs. 3 and 5 depict a number of cannula lumens that extending distally from the proximal cannula portion 16 disposed outside the patient's body to the distal cannula portion 14. The blood lumen 40 terminates at a blocked distal end at the attachment junction 24 and has a relatively large, D-shaped cross-section area. Three other smaller diameter, circular cross-section, lumens, namely a balloon inflation lumen 42, a cardioplegia delivery lumen 44, and a blood pressure lumen 46, extend through a molded section 48 of the outer sheath 18 and into the distal member 22. The lumens 42, 44, 46 can comprise lumens of separate tubes of compatible material with the outer sheath 18 that are molded into the section 48 and extend proximally and/or distally therefrom.

The proximal end of cannula body 12 extends into a Y-adapter 58 which couples the proximal ends of the lumens 40, 42, 44, 46 with an oxygenated blood connector 50, a syringe 52, a cardioplegia delivery port 54, and a pressure monitoring port 56, respectively. The Y-adapter 58 is formed with a compressible trunk 60 enclosing a trunk lumen that couples the proximal end of the blood lumen 40 with the oxygenated blood connector 50. The connector 50 is adapted to be coupled to an arterial line of an external blood oxygenator (not shown) to receive pressurized, oxygenated blood.

FIG. 1 depicts a flexible rubber hemostasis cap 78 frictionally closing the proximal end opening of the arterial connector 50 and the proximal end of a blood lumen occluder 80 extending proximally through the hemostasis valve of the hemostasis cap. The occluder 80 is formed of a flexible plastic rod long enough to extend through the blood lumen 40 to its distal end adjacent to attachment junction 24. A proximal occluder section 84 of the occluder 80 is circular in cross-section to fit tightly through the hemostasis valve. A distal occluder section 82 (shown in FIG. 3) is enlarged and shaped in a C-shape to fit tightly against the curved, inner side wall of the outer sheath 18 providing the D-shaped blood lumen 40. The C-shape distal occluder section 82 only fits in one orientation in the D-shape blood lumen so that the outer surface of the C-shape distal occluder section 82 closes the blood outlet ports 30'₁ - 30'_{N'} and/or 30₁ - 30_{N} when it is extended fully distally. The inner surface of the distal C-shape occluder section 82 does not fill the remaining space of the blood lumen 40. The C-shape minimizes the cross-section of the distal occluder section 82 so that it does not unduly stiffen the section of the cannula body that it is located in. It is intended that the occluder 80 be fully advanced as shown in FIG. 1 so that the C-shape distal occluder section 82 closes the blood outlet ports 30'₁ - 30'_{N'} and/or 30₁ - 30_{N} as the distal cannula portion 14 is advanced to the desired position of the aorta. With the proximal cannula portion 16 elevated above the distal cannula portion, a vent cap to a side port 70 of the arterial connector is then opened. Occluder 80 is then to be slowly retracted to admit blood through the successively exposed blood outlet ports 30'₁ - 30'_{N'} and/or 30₁ - 30_{N} and into the blood lumen 40 while air escapes or is purged through the side port 70. When the distal occluder section 82 is drawn against the hemostasis valve it is proximal to a section of the blood lumen in trunk 60 and the blood lumen 40 is filled with blood. The trunk 60 is then clamped by a clamp (not shown) to seal the blood lumen 40, the hemostasis cap 78 is removed from the arterial connector 50, and the occluder 80 and hemostasis cap 78 are set aside. The vent cap 70 is then closed, the arterial blood source is coupled to the arterial connector 50, and the clamp is removed from the trunk to allow arterial blood flow to pass through the blood lumen 40 and be injected through the blood outlet ports 30'₁ - 30'_{N'} and/or 30₁ - 30_{N}.

Branching tubes 62, 64 and 66 extend from connections with the proximal ends of lumens 42, 44, 46, respectively, within the Y-adapter 58 to the syringe 52, the cardioplegia delivery port 54, and the pressure monitoring port 56, respectively. The branching tubes 62, 64, 66 can be compressed to close their lumens or released to open their lumens by sliding the sliding clamps 72, 74, 76 between the compressed and open positions.

Balloon inflation lumen 42 extends into the distal member 22 and terminates at an inflation port (not shown) within the balloon lumen. Saline solution within syringe 52 can be injected or withdrawn through the branching tube 62 and the balloon inflation lumen 42 into the balloon lumen to allow it to be inflated in situ with saline solution and deflated for withdrawal of the cannula 10 by use of the syringe 52 and sliding clamp 72. The sliding clamp 72 is moved to the open position during injection and withdrawal of saline solution from the balloon lumen and is moved to the closed position when the balloon 20 is inflated to maintain it in the inflated state.

The cardioplegia lumen 44 extends through the distal member 22 and terminates at a cardioplegia outlet port set 26 distal to the balloon 20. The cardioplegia outlet port set comprises an axial lumen of the distal tip member 22 and a cross-bore that intersects it. Cardioplegia solutions can be delivered from a cardioplegia source (not shown) coupled to the cardioplegia delivery port 54 through the branching tube 64 and the cardioplegia lumen 44 and emitted axially and laterally from the cardioplegia outlet port set 26 and into the heart chambers when the sliding clamp 74 is moved to the open position. The cardioplegia passageway defined by the axial lumen of the outlet port set 26, cardioplegia lumen 44, branching tube 64 and delivery port 54 is also used to receive a previously placed guide wire to assist in introduction of the distal cannula portion to the desired site in the aorta.

The blood pressure lumen 46 extends through the distal tip member 22, through the cross-bore of the cardioplegia port set 26 and terminates at a blood pressure access port 28 that is also distal to the balloon 20 . Blood pressure measurements can be taken using a blood pressure measuring instrument (not shown) coupled to the pressure monitoring port 56 measuring the pressure of blood and cardioplegia solution that fills the lumen of the branching tube 66 and the blood pressure measurement lumen 46 when the sliding clamp 76 is moved to the open position. The pressure of the mixture cardioplegia solution and any blood in the heart chambers can be measured to ensure that the heart does not become dangerously pressurized by the cardioplegia solution.

In accordance with the present invention, the balloon 20 is asymmetric in shape (with respect to the axis of the cannula body 14 and the distal tip member 22) when inflated as shown in FIGs. 2-7. Moreover, the blood outlet ports are arranged with respect to the inflated asymmetric balloon 20 to inject oxygenated arterial blood at a maximal distance between the blood outlet port or ports and the aortic wall or other arterial vessel wall so that the force of the injected, oxygenated blood striking the same is minimized. The inflated balloon 20 is generally asymmetric as viewed in an expansion and blood injection plane 38 extending through the cannula body and distal tip member axis and the first and second sides 32 and 36 as shown in FIGs. 3 and 5. The asymmetric balloon 20 is shaped during fabrication to expand a first distance D₁ on the first side 32 of the cannula body 12 and a second distance D₂ on the second side 36 of the cannula body 12 opposite to the first side 32 when inflated. The first distance D₁ exceeds the second distance D₂, whereby the balloon 20 displaces the first side 32 away from a vascular wall that the balloon 20 bears against when inflated. Thus, the asymmetric balloon 20 is shaped to expand outward in the expansion and blood injection plane 38 more from the first side 32 of the cannula body 12 than from the second side 36 opposite to the first side 32. The asymmetric expansion displaces the first side 32 away from the side of the aorta wall that the more greatly expanded portion or lobe 21 of balloon 20 bears against when inflated. The blood outlet ports 30'₁ - 30^{'}_{N'} and/or 30₁ - 30_{N} (or a single blood outlet port) extending through the tubular outer sheath 18 and along the first side 32 proximal to the expanded balloon 20 are aligned generally with the expansion and blood injection plane 38 in the direction of distance D₂.

The asymmetric balloon shape is depicted as generally circular in FIGs. 3 and 5 and having a center point that is displaced from the center or axis of the cannula body 14 and distal tip member 22. It will be understood that the balloon 20 can be shaped in cross section in other ways, e.g. in a pear shape or an ovaloid shape or the like to accomplish the same purpose of making distance D₁ greater than distance D₂. The distance D₂ is shown as a discrete distance greater than zero in the drawings. However, it will be understood that the distance D₂ can be or can approach zero in practice.

The asymmetric balloon 20 is formed by bonding the proximal and distal end annular inner surface portions of a pre-formed tube against the cylindrical outer wall of the distal tip member 22 on either side of the distal end opening of the inflation lumen 42 within the balloon 20. The balloon 20 and tip member 22 are inserted into a holding fixture that constrains the entire circumference of the bonded annular surface portions and half the circumference of the un-bonded intermediate portion of the balloon 20. The constrained section of the balloon 20 is aligned with the cardioplegia delivery lumen 44. Air is injected through the balloon inflation lumen 42, and the holding fixture only allows the un-constrained section of the balloon 20 corresponding to lobe 21 to inflate and prevents any separation of the bonded annular surface portions from the distal tip member 22. The unrestricted portion of the balloon 20 is inflated until the tube material of the balloon wall is plastically deformed. The balloon 20 is deflated, and the distal tip member 22 and balloon 20 are removed from the holding fixture. Thus, the subsequent inflation of the balloon 20 causes it to inflate asymmetrically outward more in the plastically deformed wall section.

In the expanded views of the distal cannula portion 14 of the first embodiment shown in FIGs. 2 and 3, the plurality of blood outlet ports 30₁ - 30_{N} proximal to the balloon 20 are arranged generally in a single line extending along the first side 32 through blood injection segment length 34 and centered along the expansion and blood injection plane 38. In this embodiment, the tubular outer sheath 18 can be formed of silicone rubber having a wall thickness of about 1.0 mm and an outer diameter of about 9.0mm. The plurality of blood outlet ports 30₁ - 30_{N} can each have a diameter of about 4.0 mm and distributed uniformly spaced apart over a length 34 of about 7.5 mm.

However, it is desirable to maximize the number of blood outlet ports and their total port cross-section area to minimize blood flow restriction but without weakening the tubular outer sheath 18 so much that it might rupture or fail to provide sufficient rigidity to advance the distal cannula portion 14 over a guide wire and to the aorta. In the expanded view of the distal cannula portion 14' of the second embodiment of FIGs. 4 and 5, it can be seen that two lines 30 and 30'of blood outlet ports are depicted (with the distal occluder portion 82 removed). The two parallel lines of equidistantly spaced apart blood outlet ports 30₁ - 30_{N} and 30'₁ - 30'_{N'} are preferred to maximize blood flow, wherein the centers of the blood outlet ports 30'₁ - 30'_{N'} are staggered with respect to the centers of the blood outlet ports 30₁ - 30_{N} so that sufficient distance can be maintained between each adjacent blood outlet port to avoid unduly weakening the outer sheath 18 and/or the cannula body 12 in the distal cannula portion 14. In this case, the two lines 30 and 30' of blood outlet ports 30₁ - 30_{N} and 30'₁ - 30'_{N'} extend in parallel to one another and the longitudinal axis of the cannula body 12 in the distal cannula portion 14 are symmetrically arranged with respect to the plane 38 of asymmetric expansion of the balloon 20 extending through the axis of the cannula body 12. The first line 30 is displaced from the first side 32 and balloon expansion plane 38 extending therethrough by a first displacement angle Θ₁, and second line 30' is displaced from the first side 32 and balloon expansion plane 38 by a second displacement angle Θ₂ centered on plane 38. Preferably, the angles Θ₁ and Θ₂ are equal and range from about 45° - 60°.

In the embodiment of FIGs. 4 and 5, the tubular outer sheath 18 can be formed of silicone rubber having a wall thickness of about 1.0 mm and an outer diameter of about 9.0mm Fifteen blood outlet ports each having a diameter of about 4.0 mm are included in the two lines of blood outlet ports and are distributed uniformly spaced apart over a length 34 of about 7.5 mm. The sum of the angles Θ₁ and Θ₂ is preferably about 120°. The numbers N and N' and diameters of the blood outlet ports 30'₁ -30'_{N'} and/or 30₁ - 30_{N} are dependent on the desired oxygenated arterial blood flow rate under the applied pressure and take into account the strength of the outer tubular sheath 18.

As seen in FIGs. 6 and 7, the distal balloon 20 and at least some of the distal blood outlet ports 30₁, 30₁', 30₂, 30'₂, of cannula 10 are introduced into the aorta 108 and the remaining proximal blood outlet ports 30'₃ - 30'_{N'} and 30₃ - 30_{N} are located in the innominate artery 114. This exemplary location of cannula 10 is effected via an incision 116 in the patient's body 100 accessing the subclavian artery 102. FIG. 6 shows, in an anatomically simplified manner, access to the innominate artery 104 branching superiorly from the aortic arch of aorta 108 that is obtained through the right subclavian artery. With this structure and position, a single incision 106 is formed in the artery to introduce the cannula 10 over a previously inserted guide wire (not shown) in a manner well known in the art. A venous blood withdrawal cannula (not shown) is also inserted into the venous system through a further incision in a manner well known in the art to withdraw blood from the patient's body and to oxygenate it and pressurize it externally to return it through the arterial occlusion cannula 10 to the aorta 108.

The location of the distal cannula portion 14 can be monitored by fluoroscopy or trans-esophageal echocardiography (TEE). The TEE monitoring process is accomplished using the esophageal tube 88 and the ultrasonic waveform generator 90 in a manner well known in the art.

Before the distal cannula portion 14 is inserted through the incision 116 into the artery, the asymmetric balloon 20 is inflated with saline using the syringe 52 to observe that it does not leak and to allow air bubbles to travel proximally through the balloon inflation lumen 46 and into the syringe 52. Then, the saline is drawn out of the balloon inflation lumen 46 under a vacuum to draw the balloon 20 tightly against the distal tip member 22, and sliding clamp 72 is closed against branching tube 62 so that the distal cannula portion 14 is readied to be inserted into the incision 116. The distal cannula portion 14 and the cannula body 12 are so advanced over the guide wire extending through the cardioplegia delivery lumen 44. Then, the guide wire is withdrawn, and the blood lumen 40 is filled with the patient's blood entering the blood outlet ports or "primed" to permit connection of the blood connector with the arterial blood supply line 86 as described above.

The cardioplegia delivery lumen 44 and the blood pressure lumen 46 can also then be primed with the patient's blood by allowing air to escape the cardioplegia delivery port 54 and the blood pressure port 56, respectively. Then, cardioplegia solution can be injected through cardioplegia delivery port 54 while blood pressure is monitored. Neither operation is explicitly shown in the figures.

Air is expelled from the syringe 52, it is reattached to the branching tube 62, and the sliding clamp 72 is moved to the open position. The asymmetric balloon 20 can be inflated by injection of sterile saline contained in syringe 52 through the balloon inflation lumen 42 by manually depressing the syringe plunger. The inflation of the balloon 20 is visually monitored using fluoroscopy or TEE, and the orientation of the inflated asymmetric balloon 20 within the aorta lumen can be observed by the outline that it presents as it is inflated. The orientation can be changed by partially or completely deflating balloon 20, rotating the cannula distal portion 14 by rotating the cannula proximal portion 16, re-inflating the balloon 20 and observing the changed position. These orientation steps can be repeated until it is clear that the more expanded portion of the asymmetric balloon 20 has aligned the first side 32 away from the aorta sidewall or a vessel sidewall and in a preferred direction.

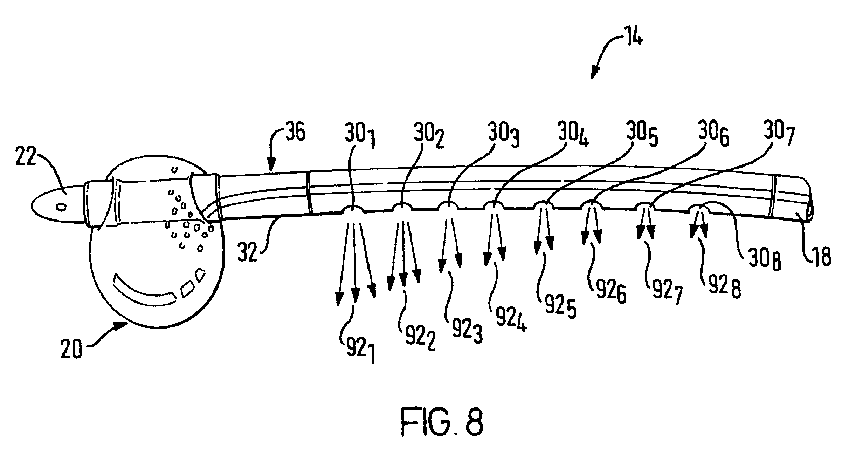

In FIGs. 6 and 7, it can be seen that when the innominate artery 114 is selected as the route for placing the balloon 20 in the aorta 108, at least a proximal sub-set of the blood outlet ports among the line 30' and/or line 30 are confined within the innominate artery 104. When the balloon 20 is inflated and the displacement of the cannula body first side 32 takes place, at least the distal most two to five distal blood outlet ports 30₁, 30₁', 30₂, 30'₂, among the line 30' and/or line 30 are displaced away from the aorta wall. However, the balloon inflation does not always appreciably displace the cannula body traversing the innominate artery. Testing has revealed that when blood is injected under pressure through the blood lumen 40 as described above, it is ejected at highest velocity from the distal most sub-set of the blood outlet ports, and the distal most jets of blood therefore exert the greatest force against any adjacent blood or anatomical structure. FIG. 8 is a schematic depiction of this disparity in ejection velocity and resulting force of the distal blood jet 92₁ injected through blood outlet port 30₁ or 30'₁ compared with each of the more proximal blood jets 92₂ - 92₈ injected through corresponding blood outlet ports. The reason for this distal bias effect is not fully understood, but may be due to the fluid viscosity of blood, the applied pressure at which it is injected into the blood lumen 40, the blood lumen cross-section size, the blood lumen surface characteristics, the cross-section areas of the blood outlet ports, and the proximity of the distal sub-set of blood outlet ports to the distal end of the blood lumen 40 blocked by the proximal end of the distal tip member 22 at attachment junction 24.

Because of this bias, it has been found that the displacement of the distal most two to five blood outlet ports among the line or lines of blood outlet ports 30' and/or 30 away from the aorta wall by the inflated asymmetric balloon 20 advantageously decreases the force of the injected blood jets striking the aorta wall. The asymmetric expansion of the balloon 20 to displace at least the distal most sub-set of the blood outlet ports 30₁, 30₁', 30₂, 30'₂ from the innominate artery where it joins the aorta and the aorta wall, is therefore advantageous, because the force diminishes with distance. The decreased force of the blood jets on the arterial or aortic wall reduces the possibility that plaque or blood clots may be dislodged, minimizing the risk of embolic complications.

In the preferred embodiment the blood outlet port lines 30, 30' extend substantially parallel to each other and also parallel to the longitudinal axis of the cannula body 12. One skilled in the art will appreciate that it is also within the scope of the invention for at least a portion of the blood outlet ports to be located elsewhere

## Claims

1. A medical cannula (10) for conducting oxygenated blood from a source exterior to a patient's body to a site in a patient's arterial vascular system, the cannula extending between a proximal cannula portion (16) for receiving the oxygenated blood and a distal cannula portion (14) for injecting the oxygenated blood into the site adjacent to a vascular wall, said cannula further comprising:
an elongated cannula body (12) extending distally from a proximal cannula body end in the proximal cannula portion to a distal cannula body end in the distal cannula portion, the cannula body formed of a tubular outer sheath (18) enclosing a blood delivery lumen (40) and a balloon inflation lumen (42) extending from said proximal cannula portion to said distal cannula portion and having a cannula body longitudinal axis;
an inflatable, asymmetric balloon (20) formed in said distal cannula portion and coupled with said balloon inflation lumen, said asymmetric balloon shaped asymmetrically with respect to the axis of the elongated cannula body (12) to expand a first distance (D₁) away from a first side (32) of the distal cannula portion and a second distance (D₂) away from a second side (36) of the distal cannula portion when inflated, the first distance exceeding the second distance, whereby the inflation of the balloon displaces the first and second sides of the distal cannula portion away from a vascular wall that the balloon bears against when inflated, and the displacement of the first side of the distal cannula portion exceeds the displacement of the second side of the distal cannula portion; and
at least one blood outlet port (30₁ - 30_{N}) extending from said blood lumen and through said tubular outer sheath (18) such that the increased displacement of the or a blood outlet port from the vascular wall caused by asymmetric expansion of the balloon away from the first side of the cannula body reduces the force of blood jets emitted from the blood outlet ports and striking the vascular wall spaced therefrom when oxygenated blood is injected through the blood lumen from the proximal cannula portion.

2. The medical cannula of Claim 1, wherein the first and second sides of the distal cannula portion are opposed to one another and fall within a blood injection plane intersecting the longitudinal axis of the cannula body, and the first and second distances extend in opposed directions in the blood injection plane.

3. The medical cannula of Claim 2, wherein the first and second distances extend in opposed first and second directions, respectively, in the blood injection plane, and the at least one blood outlet port in the first side of the distal cannula portion is aligned with the blood injection plane to emit a blood jet in the first direction.

4. The medical cannula of any preceding Claim, wherein said at least one blood outlet port (30₁ -30_{N}) comprises a plurality of blood outlet ports each extending from said blood lumen and through a first side of said tubular outer sheath (18) in said distal cannula portion and extending in a line away from said balloon.

5. The medical cannula of Claim 4 in combination with Claim 3, wherein:
each one of the plurality of blood outlet ports in the first side of the distal cannula portion is aligned with the blood injection plane to emit a blood jet in the first direction.

6. The medical cannula of any preceding claim, comprising
a first plurality of blood outlet ports (30₁ - 30_{N}) each extending from said blood lumen and through said tubular outer sheath and extending in a first line away from said balloon and in parallel with said cannula body longitudinal axis; and
a second plurality of blood outlet ports (30₁ - 30_{N}) each extending from said blood lumen and through said tubular outer sheath and extending in a second line away from said balloon and in parallel with the cannula body longitudinal axis.

7. The medical cannula of Claim 6, wherein the first plurality of blood outlet ports (30₁ - 30_{N}) in the first line are staggered in relation to the second plurality of blood outlet ports (30₁ - 30_{N}) in the second line to provide an even spacing apart and separation of the alternating adjacent blood outlet ports of the first and second pluralities of blood outlet ports.

8. The medical cannula of Claim 6 or 7, wherein the first line of the first plurality of blood outlet ports extends away from the balloon along the cannula body in the distal cannula portion in the first line displaced from the first side and the blood injection plane within a first predetermined displacement angle, and the second line of the second plurality of blood outlet ports extends away from the balloon along the cannula body in the distal cannula portion in the second line displaced from the first side and the blood injection plane within a second predetermined displacement angle.

9. The medical cannula of Claim 8, wherein the first and second displacement angles fall within an arc of about 45° to about 60° displacement from the blood injection plane intersecting the longitudinal axis of the cannula body.
